# EUROPEAN PATENT APPLICATION

(11) **EP 4 374 697 A1**
(43) Date of publication of application: **29.05.2024**
(21) Application number: 23211086.6
(22) Date of filing: 21.11.2023
(51) Int. Cl.: A01N 37/16, C07C 407/00, C07C 409/00, A61L 2/18, A01N 59/00

(54) **TWO LIQUID COMPONENT SYSTEM FOR OBTAINING A SOLUTION OF PERACETIC ACID AND TEA TREE OIL**

(30) Priority: 22.11.2022 IT 202200023985
(71) Applicant: Adranox Srl, 46100 Mantova (MN) (IT)
(72) Inventor: BACCHI, Raffaele, I-37017 Lazise (VR) (IT)
(74) Representative: Dragotti & Associati S.R.L.

(57) **Abstract**

The present invention relates to a two liquid component system and a process for obtaining a solution of peracetic acid and Tea Tree Oil to be used as is or to be diluted, as well as the use of this solution as a biocide in the field of disinfection, chemical sterilization, and in other fields where peracetic acid is used together with Tea Tree Oil due to their biocidal properties.

## Description

### Background of the invention

The present invention relates to a two liquid component system and a process for obtaining a solution of peracetic acid and Tea Tree Oil (TTO) to be used as is or to be diluted, as well as the use of this solution as a biocide in the field of disinfection, chemical sterilization, and in other fields where peracetic acid is used together with Tea Tree Oil due to their biocidal properties.

### State of the art

To date, the sensitivity of many materials, such as endoscopes, has led to the use of various high-level disinfectant products, among which, until recently, the most widespread were aldehyde derivatives (Glutaraldehyde, Orthophthalaldehyde, Glyoxal, and various other derivatives). Despite having an excellent biocidal spectrum, such compounds show very strong limitations in their safe use for operators as, in addition to being very volatile, they are also potentially dangerous for human health as well as not environmentally friendly. Various products based on peracetic acid have therefore been introduced onto the market, both ready-to-use, at various concentrations, and in the form of two components (Generator and Activator), for the extemporaneous production of peracetic acid after activation.

From a chemical point of view, peroxyacids (or peracetic acid) are peroxides of organic acids of general formula (I)

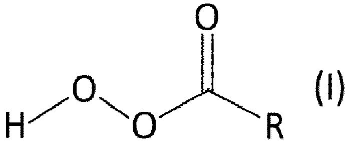

Where R is an alkyl or aryl group.

In particular, peracetic acid is the peroxyacid of acetic acid, where the R group corresponds to a methyl group (-CH₃), which is omitted by convention (II) Peracetic acid

(CH₃COOOH)

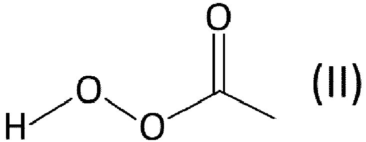

Peracetic acid appears as a liquid with an acrid odor. It is marketed in aqueous solutions having concentrations of approximately 1.5%, 5%, 15%, and 35% w/w. Due to the peracetic acid properties of being a strong oxidant and a broad-spectrum biocide, these solutions are used as bleaching, descaling agents, and especially as disinfectants and sterilizers in the medical-surgical field, typically for the disinfection of thermosensitive devices and equipment (*e.g*. endoscopes) as well as for environmental surfaces in surgery rooms, *etc.*

Depending upon their concentration, peracetic acid solutions are, however, irritating and corrosive to the skin, as well as combustive at very high concentrations, so concentrated solutions should be appropriately diluted before being used. Dilution, however, while solving on the one hand the problem of dangerous handling and transportation, on the other reduces peracetic acid stability.

At an industrial level, peracetic acid is prepared by reacting hydrogen peroxide (commonly called oxygenated water) with acetic acid according to the following synthetic scheme:

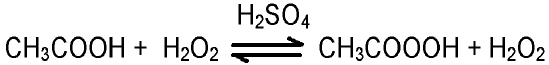

The above reaction leads to the formation, at the equilibrium, of a mixture of reactants and products. To favor the rapid formation and stability of peracetic acid in solution, the addition of an acid catalyst, such as a strong inorganic acid, such as sulfuric acid, is provided for. It follows that commercially available peracetic acid solutions are strongly acidic and, therefore, despite their stability, they are particularly aggressive towards various materials to be treated.

At concentrations ≥ 5%, peracetic acid is corrosive and harmful by inhalation, ingestion, and contact with the skin. This danger characteristic also affects transportation by road, rail, sea, and air. In fact, specific restrictions for their transport, and also with regard to packaging and labelling, are envisaged for corrosive and oxidizing substances or preparations, as well as for organic peroxides.

Another method for preparing diluted, and therefore ready-to-use, peracetic acid solutions consists in the reaction between N,N,N',N'-tetraacetylethylenediamine (TAED) and an alkaline adduct of hydrogen peroxide, such as sodium perborate or sodium percarbonate, in which hydrogen peroxide reacts according to the following synthetic scheme:

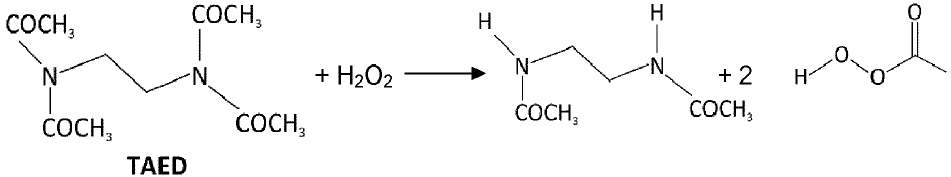

Such reaction occurs at a basic pH, which, besides allowing a rapid nucleophilic attack to the carbonyl carbon of the acetyl group, favors the release of active oxygen, with a consequent rapid decrease in the concentration of peracetic acid. Furthermore, another drawback of this method is represented by the presence of solid particles in suspension that can clog the tiny channels of endoscopes during decontamination and/or disinfection of fiber optic equipment with these solutions.

Other methods of preparing peracetic acid solutions are described in the following patent documents.

The patent EP0953283B9 teaches the extemporaneous preparation of a hydroalcoholic solution of peracetic acid by mixing an aqueous solution of O-acetylated or N-acetylated compounds with a hydrogen peroxide-based activator. The aqueous environment in which the O-acetylated or N-acetylated compounds are dispersed inevitably makes them unstable as they undergo hydrolysis over time.

The patent EP1077606B1 describes, instead, the preparation of an antimicrobial composition and its use for the disinfection of surfaces and/or surgical instruments, endoscopes, circuits, *etc.,* by mixing an oxygen-generating liquid base, such as hydrogen peroxide, and a liquid generator of acetyl groups consisting of N-acetylcaprolactam. To accelerate the perhydrolysis reaction, the hydrogen peroxide solution is maintained at a neutral-basic pH by adding a basic and nucleophilic compound, such as sodium or potassium hydroxide or an alcoholamine (for example, monoethanolamine, diethanolamine, triethanolamine), or a basic adduct of hydrogen peroxide (for example, sodium perborate or percarbonate), which could not be previously mixed with N-acetylcaprolactam since it would become degraded. Under these conditions, however, hydrogen peroxide is particularly unstable, as it undergoes dismutation into water and oxygen, and the latter escapes the solution in gaseous form, thus reducing the concentration thereof, while lowering the pH value of the solution itself. The release of gaseous oxygen also poses problems from a practical point of view, since it is necessary that the oxygen donor solution is stored and transported in a cool environment, and in containers equipped with a vented cap to avoid excessive swelling of the packaging itself.

The patents ITVR20100033 and EP2388246B1 describe the introduction of an amino compound, claiming certain properties thereof, such as its basic character: upon mixing, the immediate increase in pH would allow peracetic acid to be rapidly generated, thus reaching a high concentration of the same in a period of time of the order of a few minutes.

However, the presence of the tertiary amine, which is potentially dangerous due to flammability, toxicity and corrosivity, makes the handling of the product unsafe, as well as leading to a notable peak, in ppm concentration, of peracetic acid, which in the first days after activation could be harmful to the materials it comes into contact with, due to the characteristics described above.

Therefore, the need is felt to have a system for extemporaneously preparing a concentrated or diluted solution of peracetic acid together with Tea Tree Oil, a system that is stable over time and not dangerous for handling and transportation thereof, as well as having wide compatibility with the material.

### SUMMARY OF THE INVENTION

The main object of the present invention is to provide a two liquid component system, for extemporaneously preparing a solution of peracetic acid together with Tea Tree Oil (TTO), which retains its chemical-physical characteristics substantially unchanged for long periods of time after activation (slow release system) and avoid having peak concentrations of developed peracetic acid (in ppm), which can cause damage to the materials.

Another object of the present invention is to provide a two liquid component system for preparing a solution of peracetic acid and Tea Tree Oil, a system which is easy and safe to handle and transport.

A further object of the present invention is to provide a two liquid component system whose chemical composition is such as to allow an acceptable *in situ* preparation time of a peracetic acid solution, which can grow over time with lower concentration peaks and with greater stability once activated.

Another object of the present invention is to provide a process for obtaining, at the time of use, a diluted and ready-to-use solution of peracetic acid and Tea Tree Oil.

A further object of the present invention is to make available a diluted and stable solution of peracetic acid and Tea Tree Oil for direct use as a biocide in the chemical disinfection and/or sterilization processes of surgical/endoscopic instruments and/or environmental surfaces, especially in the medical field. According to a first aspect of the present invention, a system is provided consisting of a first and a second liquid component characterized in that
- said first liquid component comprises at least one donor of at least one acetyl group selected from N-acetylated and O-acetylated compounds, and Tea Tree Oil;
- said second liquid component comprises at least one oxygen generator.

According to a further aspect of the present invention, a process is provided for extemporaneously preparing a solution of peracetic acid and Tea Tree Oil comprising the step of mixing the two liquid components of the system according to the invention.

According to a further aspect of the present invention, the use of the system according to the invention as a biocide in the chemical disinfection and/or sterilization processes of surgical/endoscopic instrumentation and/or environmental surfaces, especially in the medical field, is provided.

Further objects and advantages of the present invention will appear better from the detailed description of certain currently preferred embodiments thereof, illustrated below for illustrative and non-limiting purposes.

### BRIEF DESCRIPTION OF THE FIGURES

- Figure 1 shows a comparison in terms of stability over time of the solutions of Example 1 (reference - NR3, no TTO) and Example 3 (invention - TTO, no NR3).
- Figure 2 shows a comparison in terms of stability over time of the solutions of Example 2 (reference - no TTO) and Example 3 (invention - TTO).
- Figure 3 shows the diagrams relating to the variation in pH over time of the peracetic acid solution obtained in Example 1 **(Diagram A),** and the variation in concentration of peracetic acid over time **(Diagram B).**

### DEFINITIONS

Unless otherwise defined, all terms of the art, notations and other scientific terminology used herein are intended to have the meanings commonly understood by those skilled in the art to which this disclosure pertains. In some cases, terms with commonly understood meanings are defined herein for clarity and/or for ready reference; thus, the inclusion of such definitions in the present disclosure should not be construed to represent a substantial difference over what is generally understood in the art.

Within the scope of this description and in the subsequent claims, all numerical quantities indicating amounts, parameters, percentages, and so on, are to be understood as preceded in all circumstances by the term "about" unless otherwise indicated. Furthermore, all ranges of numerical quantities include all possible combinations of the maximum and minimum numerical values and all possible intermediate ranges, in addition to those specifically indicated below. The term **"C₁-C₁₀ alkyl"** refers herein to a branched or linear hydrocarbon containing 1 to 10 carbon atoms. Examples of C₁-C₁₀ alkyl groups include, but are not limited to, methyl, ethyl, n-propyl, isopropyl, n-butyl, sec-butyl, isobutyl, tert-butyl, n-pentyl, n-hexyl.

The term **"aryl"** refers herein to aromatic mono- and poly-carbocyclic(s) ring systems, where individual carbocyclic rings in poly-carbocyclic ring systems can be fused or attached to each other through a single bond. Suitable aryl groups include, but are not limited to, phenyl, naphthyl and biphenyl.

### DETAILED DESCRIPTION OF THE INVENTION

A first aspect of the invention relates to a system consisting of a first and a second liquid component characterized in that
- said first liquid component comprises at least one donor of at least one acetyl group selected from N-acetylated and O-acetylated compounds, and Tea Tree Oil;
   - - said second liquid component comprises at least one oxygen generator. Advantageously, the system of the present invention prevents high concentrations of peracetic acid from being obtained in the first few minutes (thus avoiding a concentration peak which can be harmful both to the user's health and deleterious for the treated materials).

Moving said peak further in time advantageously translates into an extended stability of the activated solution.

In particular, the currently preferred N-acetylated compounds include N,N,N',N'-tetraacetylethylenediamine, tetracetylglycoluryle, one or more (C₄-C₁₀)-N-acetylactams, preferably N-acetylcaprolactam, and one or more N-acetylimides having a number of carbon atoms greater than or equal to 4, such as for example N-acetylsuccinimide, N-acetylphthalimide, and 2-n-nonyl-N-acetylsuccinim ide.

While the currently preferred O-acetylated compounds include acetic anhydride, acetylsalicylic acid, and acetate esters of sugars, such as glucose pentaacetate, sucrose octaacetate and mannitol hexaacetate.

A use of N-acetylated or O-acetylated compounds as activators of peroxide groups was described, for example, in patent documents WO95/20876, US4128494 and US5654269, which describes compounds having the following general formula:

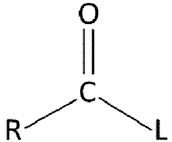

Where R is a methyl and L is a good leaving group having a nitrogen or oxygen atom directly bonded to the carbonyl carbon atom.

As is known, in fact, a N-acetyl or O-acetyl group has electrophilic chemical properties, which makes it susceptible to attack by a nucleophile, *i.e*. a chemical species capable of donating an unshared electron pair, with consequent breaking of the amide or ester bonds and the release of the acetyl group by the donor molecule, respectively.

Examples of molecules and ions that behave as nucleophiles are water (H₂O), ammonia (NH₃), alcohols (ROH), ethers (ROR), primary (RNH₂), secondary (R₂NH) and tertiary (R₃N) amines, hydroxyl ion (OH⁻), alcoholate (RO⁻) and thiolate (RS⁻) ions. The nucleophilic character of these species depends on various factors, among which the most relevant are pKa value (negative logarithm of the acid dissociation constant of a protonated chemical species), electronegativity, presence of electron donating or electron withdrawing groups or atoms, steric hindrance, and nature of the solvent. As regards steric hindrance, the presence in a molecule or ion of voluminous, so-called "bulky", substituents, has a negative effect on its nucleophilic character, and therefore reduces its tendency to react with an electrophilic species.

Factors such as electronegativity and the presence in a molecule of electron withdrawing or electron donating atoms and/or groups also influence the electrophilic properties of an electrophilic chemical species, so, for example, the more its carbonyl carbon is affected by the electron withdrawing effect exerted by carbonyl groups present in a vicinal position, the more N-acetyl or O-acetyl group is electrophilic.

The use of Tea Tree Oil (TTO), in addition to its antibacterial, antifungal, antiinflammatory and antiparasitic properties, has other advantages, especially when it interacts with organic peracids as in the present invention.

The presence in the system of the present invention of an essential oil, in this case Tea Tree Oil, is advantageous both for its proven anti-microbial activity and for its stabilizing nature, an intrinsic property deriving from the high concentration of terpene alkenes in it.

Tea Tree Oil is in fact made up mostly of terpene alkenes, such as α-terpinene and γ-terpinene and, as will be illustrated in Example 3 (extemporaneous preparation of a peracetic acid solution - non-amino derivative), the multiple double bonds provided by this essential oil allow the formation of a sort of "slow-release epoxy buffer", aimed at dampening the peracetic acid formation and degradation rates, with important advantages in terms of compatibility with materials.

The system according to the present invention is aimed at the preparation of a stable solution of peracetic acid and Tea Tree Oil, which is made, at time of use, according to the process of the present invention which essentially involves reacting, by mixing, the two system components.

In particular, the oxygen generator is stored in an acidic aqueous solution and in a mole concentration higher than that of the donor of one or more acetyl groups, so that the molar ratio between the two components is always > 1.

Preferably, the oxygen generator according to the present invention comprises an acidic aqueous solution (constant pH and < 5 U pH) of hydrogen peroxide at a concentration by weight between 0.01% and 90%, and preferably between 3.0% and 35 %.

Under these conditions, the aqueous hydrogen peroxide solution is stable and can be stored for at least two years at room temperature (approximately 25°C) and for 14 days at 54°C without undergoing chemical-physical alterations, as determined by the "MT 46 Accelerated Storage Tests by Heating" CIPAC method.

In a preferred embodiment, said at least one donor of at least one acetyl group is present at a percentage concentration equal to or greater than 80% w/w.

In a further preferred embodiment, Tee Tree Oil is present at a percentage concentration between 0.001% and 10% w/w, preferably between 0.002% and 0.010% w/w.

The optimal concentration resulted to be 0.002%, already an active concentration (TTO is, in fact, active even at minimal concentrations). Higher concentrations were also tested (0.005% and 0.010%) which demonstrated the same activity but the onset of a very strong odor (typical of TTO, in fact).

In a preferred embodiment, before the reaction phase, it is possible to add one or more chemical additives selected from alcohols, for example ethyl and isopropyl alcohol, surfactant agents, dispersing agents, coloring substances, anticorrosive agents, diols and polyols, to one of the two components of the system.

Such chemical additive(s), if added to the mixture according to the present invention, should be anhydrous and devoid of nucleophilic character to avoid degradation of the acetyl group donor.

The solutions of peracetic acid and Tea Tree Oil, extemporaneously prepared according to the process of the present invention, can be both aqueous solutions and hydroalcoholic solutions. Furthermore, such solutions, prepared according to the process of the present invention, are used in cold chemical disinfection and sterilization processes of medical-surgical instruments and/or equipment, filtration systems, as well as environmental surfaces. Medical-surgical instruments and/or equipment concern in particular thermosensitive fiber optic equipment, such as rigid or flexible endoscopes and transesophageal probes.

Furthermore, the extemporaneous solutions of peracetic acid, prepared according to the process of the present invention, can be prepared, diluted, and used directly in automated washing machines, for example endoscope washers and surgical instrument washer or dialysis equipment.

N-acetylated and O-acetylated chemical compounds, Tea Tree Oil, oxygen generator and additive(s), which can be used in the present invention, are commercially available, for example by Sigma-Aldrich Inc., BASF Italia S.r.l, *etc..*

A particularly preferred system according to the present invention consists of:
a. Liquid oxygen generator containing stabilized hydrogen peroxide at a concentration between 0.1% and 90% by weight, preferably between 3.5% and 10% by weight;
b. Liquid activator comprising at least one donor of at least one acetyl group selected from N-acetylated and O-acetylated compounds, and Tea Tree Oil consisting, in w/w, of:
   i. N-acetylcaprolactam: ≥ 80%, preferably between 80.5% and 90%,
   ii. Tee Tree Oil: 0.001% to 10%, preferably between 0.002% and 0.010%,
   iii. Alcohol: 5% to 50%,
   iv. Eco-friendly chelator: 0.1% to 10%,
   v. Reaction adjuvant: 0.1% to 10%,
   vi. Anticorrosive agent: 0.1% to 30%.

Examples of additives that can be used in the present invention are:
- ethyl alcohol, isopropyl alcohol, or mixtures thereof;
- chelators selected from GLDA (L-glutamic acid-N,N diacetic acid), EDTA (ethylenediaminetetraacetic acid), IDS (Sodium iminodisuccinate) or NTA (nitrilotriacetic acid);
- adjuvants selected from carboxylic acids, preferably acetic acid;
- anticorrosive agents selected from azoles and thiazoles, preferably benzotriazole.

A second aspect of the present invention relates to a process for extemporaneously preparing a solution of peracetic acid and Tea Tree Oil comprising the step of mixing the two liquid components of the system according to the invention.

In a preferred embodiment, the final activated solution of peracetic acid has a concentration comprised between 100 ppm (parts per million) and 2500 ppm (parts per million).

In a further preferred embodiment, the solution of peracetic acid and Tea Tree Oil obtained by the process according to the invention is diluted with water before use.

The mixing step, according to the process of the present invention, can take place at any temperature between 0°C and 80°C, preferably between 10°C and 60°C.

In a preferred embodiment, the process according to the present invention allows the preparation of peracetic acid solutions at any concentration between 0.00001% and 40% w/w or w/v, preferably between 0.01% and 35% w/w or w/v.

More specifically, by varying the concentration of hydrogen peroxide used in the process according to the present invention as well as its molar ratio to the acetyl group donor, various peracetic acid solutions, both diluted and concentrated, can be obtained.

Diluted solutions of peracetic acid have a concentration preferably between 0.0001% and 0.30% by weight.

Once the activator and generator are mixed, TTO has a concentration of at least 0.00002% by weight, sufficient to generate the epoxide system. In fact, the inventors have verified that this "slow release" system does not occur at lower concentrations.

A third aspect of the present invention relates to the use of the system of the present invention as a biocide in the chemical disinfection and/or sterilization processes of surgical/endoscopic instrumentation and/or environmental surfaces, especially in the medical field.

By way of non-exhaustive example only, the present invention may be used on surgical, endoscopic instrumentation, optic fibers, and other medical devices in the medical field, but not only.

Furthermore, the present invention may be used in all professional fields where the use of peracetic acid is required to perform high-level disinfection or cold chemical sterilization of surfaces and/or objects.

Below is a comparison between a peracetic acid solution developed by a classical activator containing the reference amine (Example 1), a peracetic acid solution developed by an activator not containing the reference amine (Example 2) and one developed by an activator not containing the amine, but Tea Tree Oil according to the invention (Example 3).

### EXAMPLE 1 (Reference Solution)

### Extemporaneous preparation of a (amino-derivated) peracetic acid solution according to Example 1 of the patent EP238824681

A diluted solution of peracetic acid was prepared according to the following reaction:

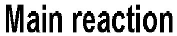

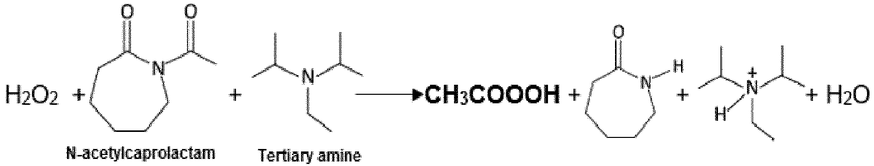

A 3% by weight hydrogen peroxide solution was prepared by diluting 85.71 g of 35% w/w hydrogen peroxide with enough purified water to obtain 1000 g, and the pH at 20°C was determined. 6.4 g of a mixture containing N-acetylcaprolactam (6.20 g) and N,N,N-diisopropylethylamine (0.20 g) were prepared separately, and the mixture obtained was added to one liter of 3% w/w hydrogen peroxide solution to obtain a solution of peracetic acid having a concentration, in the first hours after activation, comprised between 0.09% and 0.15% by weight.

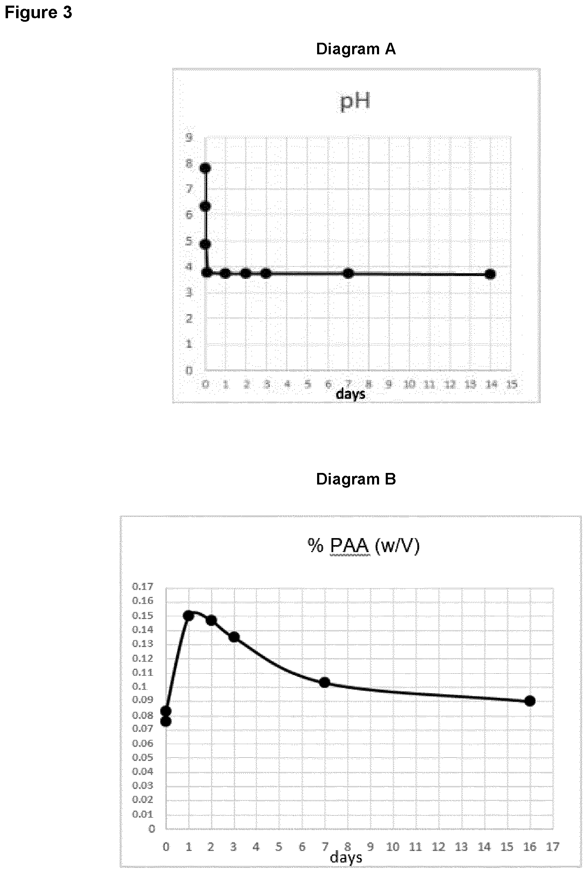

Both the variation in the pH of the peracetic acid solution thus obtained and the variation in concentration of peracetic acid over time were determined, and the results are shown in Table 1 and in diagrams A and B in **Figure 3****.**

**Table 1**

| **Reagents** | **% w/w** | **Chemical-physical characteristics** | **Amount (g)** |
|---|---|---|---|
| *Oxygen generator* | | | |
| Hydrogen peroxide | 3.00 | pH = 3.80 - 4.20 U pH at 20°C | 1000 |
| Purified water q.s. to | 100 | | |
| *Composition of an acetyl group donor and tertiary amine* | | | |
| N-acetylcaprolactam | 96.875 | | 6.4 |
| N,N,N-diisopropylethylamine | 3.125 | | |

| **Products** | | | |
|---|---|---|---|
| *Peracetic acid solution* | | | |
| Hydrogen peroxide | 2.91 | pH = 7.80 (t0) - 6.33 (t5min) | 1006.4 |
| Peracetic acid | 0.15-0.09 | 4.85 (t30min) - 3.75 (t120min) - 3,70 (t14d) | |
| Caprolactam | 0.46 | Stability > 14d at 25°C for Concentrations > 0.09% w/V | |
| N,N,N-diisopropylethylammonium | 0.02 | | |
| Purified water q.s. to | 100 | | |

### EXAMPLE 2 (reference solution)

### Extemporaneous preparation of a peracetic acid solution according to the patent EP095328389

A diluted solution of peracetic acid was prepared according to the following reaction:

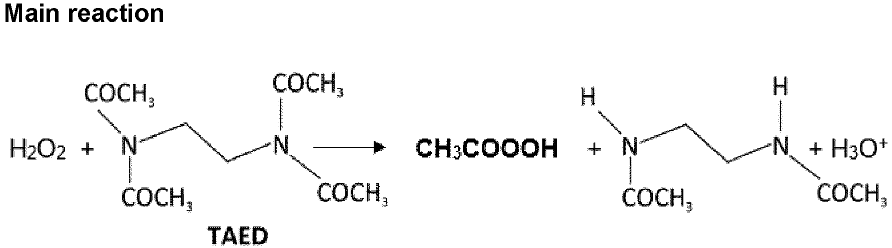

**Table 2**

| **Reagents** | **% w/w** | **g for 250g of solution** |
|---|---|---|
| Distilled water | 89.100 | 22.750 |
| TAED | 0.400 | 1.000 |
| Glacial acetic acid | 10.000 | 25.000 |
| Concentrated sulfuric acid | 0.500 | 1.250 |

The components were weighed separately. To solubilize tetraacetylethylenediamine (TAED), the water was heated to about 70-80°C with stirring. After cooling the solution to 20°C, glacial acetic acid and concentrated sulfuric acid were added, taking every necessary precaution with the latter.

After the addition of the components, further stirring was carried out for 15 minutes and the product was allowed to rest for 10 minutes.

The activator used was 35% w/w hydrogen peroxide in the amount of 5 g per 250 g of stock solution, therefore 2 g per 100 g of stock solution.

### EXAMPLE 3 (Solution according to the invention)

### Extemporaneous preparation of a solution of peracetic acid (not amino-derived) and Tea Tree Oil

A dilute solution of peracetic acid was prepared according to the following reaction:

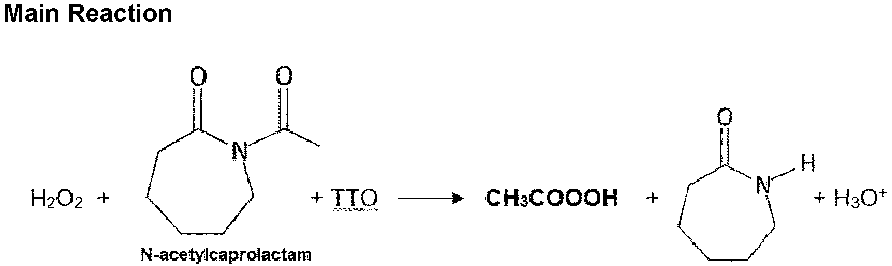

**Table 3**

| **Reagents (ACTIVATOR*)** | **% w/w** | **g for 100g of solution** |
|---|---|---|
| N-acetylcaprolactam | 80.500 | 80.500 |
| Ethanol | 15.498 | 15.498 |
| GLDA | 0.500 | 0.500 |
| Glacial acetic acid | 0.500 | 0.500 |
| Benzotriazole | 3.000 | 3.000 |
| Tea Tree Oil | 0.002 | 0.002 |

| **Reagents (GENERATOR*)** | **% w/w** | **g for 1000g of solution** |
|---|---|---|
| Distilled water | 96.400 | 964.00 |
| Hydrogen peroxide | 3.600 | 36.00 |

| | | |
|---|---|---|
| **N.B: in this example, ACTIVATOR and GENERATOR are exchanged* | | |

The components were weighed separately. The solutions were prepared cold, with the help of a magnetic stirrer in the case of the activator, adding the components in the order of Table 3. After the addition of the components, the solution was kept under stirring for 10 minutes.

The generator used was 3.6% hydrogen peroxide, as shown in Table 3.

Next, the activation of the solution was performed by taking 10 mL of activator and adding them to 990 mL of generator, thus obtaining a final (activated) solution at 1% v/v of activator.

There is also an important accessory reaction linked to the presence of Tea Tree Oil.

Tea Tree Oil is mainly produced from *Melaleuca alternifolia (Myrtaceae* family). It contains monoterpenes and related alcohols, among which terpinen-4-ol is the most abundant constituent (40%). ISO 4730 establishes acceptability ranges for 15 components, and TTO should fall within this ranges in order to be legally marketed as such. Originally obtained from plants that developed in nature, the oil is now produced from *M. alternifolia* plantations in many countries, including Australia, Kenya, and China. This essential oil was historically used as a topical antiseptic in Australia, based on its documented broad-spectrum anti-microbial activity. To date, it is mainly used in the field of hand hygiene and skin antisepsis, but also in products for cleaning and disinfecting surfaces (Brophy JJ et al. J Agric Food Chem. 1989;37:1330-1335; ISO 4730:2017: Essential oil of Melaleuca, Terpinen-4-ol Type (Tea Tree Oil), Geneva, Switzerland; Carson CF et al. Clin Microbiol Rev. 2006;19:50-62).

Since Tea Tree Oil is largely made up of terpinen-4-ol, α-terpinene and γ-terpinene, these are the main molecules whose double bonds should be considered:

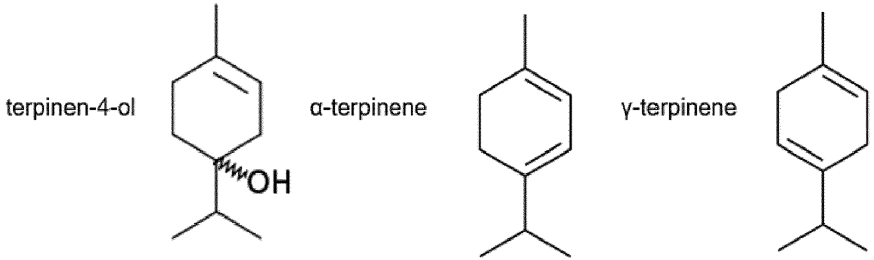

These double bonds act as useful sites for the epoxidation reaction, typical of alkenes, with organic peracids, according to the following reaction scheme:

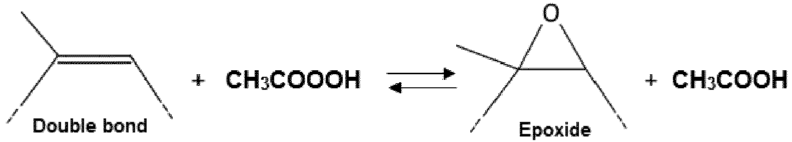

This sort of *"slow-release epoxy buffer"* that is formed thus allows a dampened and extended peracetic acid release rate over time. This allows us to avoid an unnecessarily high concentration peak, which would otherwise be deleterious for the materials in contact, especially for prolonged immersion falling precisely in the time range corresponding to the peak.

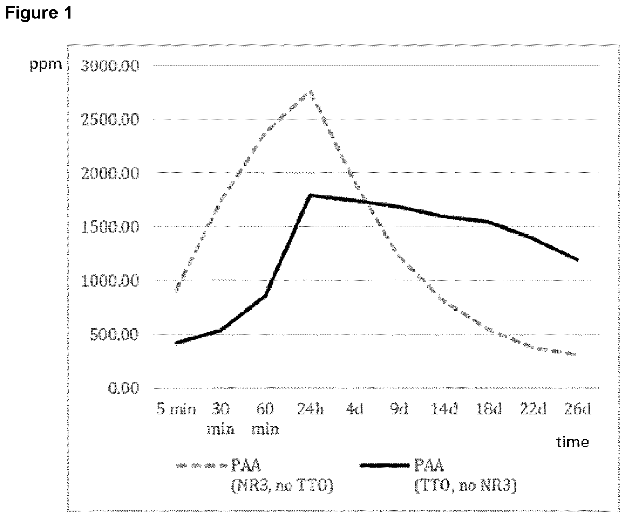

**Figure 1** shows the comparison between the 2 solutions (Examples 1 and 3), both derived from the same oxygen generator, *i.e*. a 3.0% H₂O₂ solution (10 Volumes of O₂), in Example 1 and 3.6% (12 volumes of O₂) in the invention (Example3):

**Table 4**

| **t** | **PAA (no TTO, NR3) [ppm]** | **PAA (TTO, no NR3) [ppm]** |
|---|---|---|
| 5 min | 909.00 | 420 |
| 30 min | 1750.00 | 540 |
| 60 min | 2381.00 | 860 |
| 24h | 2769.00 | 1800 |
| 4d | 1922.00 | 1750 |
| 9d | 1232.00 | 1690 |
| 14d | 811.00 | 1600 |
| 18d | 544.00 | 1550 |
| 22d | 379.00 | 1400 |
| 26d | 314.00 | 1200 |

The following is apparent from Figure 1 :
- the dotted line consists of the addition of an activator containing 60% N-acetylcaprolactam and a tertiary amine (reference - NR3, no TTO): this solution claims a stability of 14 days from activation; furthermore, the presence of the amine accelerates the initial peracetic acid formation, causing it to reach concentrations of over 900 ppm after just 5 minutes. But as mentioned, this initial acceleration, according to experimental laboratory data, while being on the one hand useful for an almost immediate use of the product after activation, certainly makes it more aggressive (peracetic acid ppm keeps increasing even beyond the threshold of 2000 ppm in the first 24 hours after activation) and not stable beyond 14 days, with a steeper slope of the decay curve; with consequent risk for the materials and for the safety of the operators, depending on the intrinsic characteristics of the peracetic acid described above.
- the black line consists in the addition of an activator containing 80.5% N-acetylcaprolactam and Tea Tree Oil (subject-matter of the present invention - TTO, no NR3): the threshold of 500 ppm, necessary to be able to claim its anti-microbial activity, is reached just before 30 minutes, but this solution claims stability for up to at least 18 days after activation.

The absence of the amine in the solution of the present invention will prevent high concentrations of peracetic acid from being obtained in the first few minutes, but will allow two advantages: the first consists in reducing the concentration peak, thus avoiding unnecessarily accentuated effects, when not deleterious, for the materials; the second consists in moving said peak further away in time, with consequent extension of the activated solution stability to over 18 days of stability above the threshold of 500 ppm.

The activated solution of the present invention has in fact unexpectedly shown a stability superior to that of the reference solution (EP2388246B1).

Lastly, it should not be ignored that the absence of a compound such as a tertiary amine allows for significantly safer handling of the product, as it is potentially dangerous due to flammability, toxicity and corrosivity.

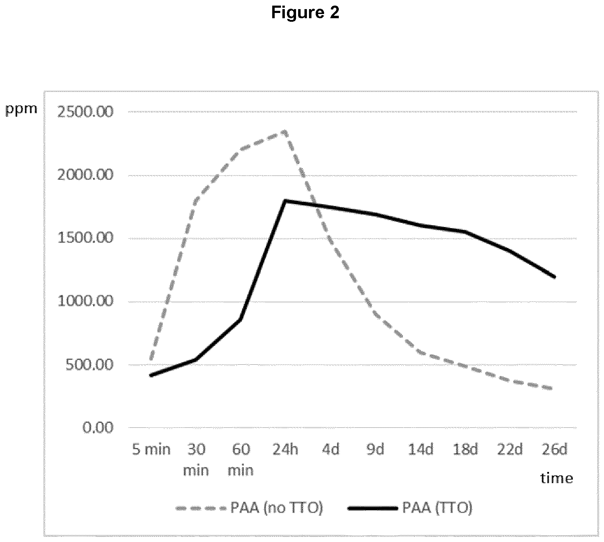

**Figure 2** shows instead the comparison between the 2 solutions not containing the amine (Examples 2 and 3), both derived from the same oxygen generator, *i.e.* a 2.0% H₂O₂ solution, in Example 2 and 3.6% (12 volumes of O₂) in the invention (Example 3):

**Table 5**

| **t** | **PAA (no TTO) [ppm]** | **PAA (TTO) [ppm]** |
|---|---|---|
| 5 min | 550 | 420 |
| 30 min | 1800 | 540 |
| 60 min | 2200 | 860 |
| 24h | 2350 | 1800 |
| 4d | 1490 | 1750 |
| 9d | 900 | 1690 |
| 14d | 600 | 1600 |
| 18d | 490 | 1550 |
| 22d | 370 | 1400 |
| 26d | 310 | 1200 |

The following is apparent from Figure 2:
From the experimental laboratory data, after adding the ACTIVATOR (containing the acetyl donor) to the GENERATOR (containing peroxide), the reference solution not containing Tea Tree Oil certainly reaches the biocidal activity in shorter times, but this gives rise to unnecessarily high ppm, which can be deleterious. Furthermore, its decay is also faster, making the solution ineffective in the short term.

Thanks to the epoxy buffer, the solution containing Tea Tree Oil according to the invention helps to avoid both problems, with the only need to wait a few minutes to reach activation.

The activated solution of the present invention has in fact unexpectedly shown superior stability even compared to that of the reference solution (EP0953283B9).

Naturally, a person skilled in the art will be able to make modifications and variations to the invention described above in order to satisfy specific and contingent application needs; variations and modifications still falling within the scope of protection as defined by the subsequent claims.

## Claims

1. System consisting of a first and a second liquid component **characterized in that**
- said first liquid component comprises at least one donor of at least one acetyl group selected from N-acetylated and O-acetylated compounds, and Tea Tree Oil;
- said second liquid component comprises at least one oxygen generator.

2. System according to claim 1, **characterized in that** said N-acetylated compounds are selected from the group comprising N,N,N',N'-tetraacetylethylenediamine, tetracetylglycoluryle, (C₄-C₁₀)-N-acetylactam, N-acetylimide having a number of carbon atoms greater than or equal to 4, and mixtures thereof, preferably said (C₄-C₁₀)-N-acetylactam is N-acetylcaprolactam, and said N-acetylimide is selected from N-acetylsuccinimide, N-acetylphthalimide, and 2-*n*-nonyl-N-acetylsuccinimide.

3. System according to claim 1 or 2, **characterized in that** said O-acetylated compounds are selected from the group comprising acetic anhydride, acetylsalicylic acid, sugar acetate esters, and mixtures thereof, preferably said sugar acetate esters comprise glucose pentacetate, sucrose octacetate and mannitol hexacetate.

4. System according to any one of the preceding claims, **characterized in that** said Tea Tree Oil comprises terpene alkenes and terpene alcohols, preferably α-terpinene, γ-terpinene, and terpinen-4-ol.

5. System according to any one of the preceding claims, **characterized in that** the molar ratio between said oxygen generator and said at least one donor of at least one acetyl group is greater than 1.

6. System according to any one of the preceding claims, **characterized in that** said oxygen generator comprises an acidic aqueous solution of hydrogen peroxide at a concentration between 0.01% and 90% by weight, preferably between 3.0% and 35% by weight.

7. System according to any one of the preceding claims, **characterized in that** said at least one donor of at least one acetyl group is present at a concentration equal to or greater than 80% w/w.

8. System according to any one of the preceding claims, **characterized in that** said Tee Tree Oil is present at a concentration between 0.001% and 10% w/w, preferably between 0.002% and 0.010% w/w.

9. System according to any one of the preceding claims, **characterized in that** at least one chemical additive is added to one of the two components, said at least one chemical additive being selected from alcohols, surfactants, dispersing agents, dyes, anticorrosive agents, diols, and polyols.

10. Process for the extemporaneous preparation of a solution of peracetic acid and Tea Tree Oil comprising the step of mixing the two liquid components of the system according to any one of the preceding claims.

11. Process according to claim 10, **characterized in that** said solution has a peracetic acid concentration between 100 ppm and 2500 ppm.

12. Process according to claim 10 or 11, **characterized in that** said solution of peracetic acid and Tea Tree Oil is diluted with water, before use.

13. Process according to any one of claims 10 to 12, **characterized in that** said mixing step is carried out at a temperature between 0°C and 80°C, preferably between 10°C and 60°C.

14. Process according to any one of claims 10 to 13, **characterized in that** said solution of peracetic acid and Tea Tree Oil is an aqueous or hydroalcoholic solution.

15. Use of the system according to any one of claims 1 to 9, as a biocide in the disinfection and/or chemical sterilization processes of surgical/endoscopic instrumentation and/or environmental surfaces, preferably in the medical field.
